# EUROPEAN PATENT APPLICATION

(11) **EP 1 980 289 A2**
(43) Date of publication of application: **15.10.2008**
(21) Application number: 08013262.4
(22) Date of filing: 20.01.2006
(51) Int. Cl.: A61M 25/10, A61M 25/00

(54) **Catheter having a soft distal tip**

(30) Priority: 21.01.2005 US 646118
(62) Divisional of application: 06001187.1
(71) Applicant: Abbott Laboratories Vascular Enterprises Limited, Dublin 2 (IE)
(72) Inventor: Jeffrey, Andrew, 72074 Tuebingen (DE); Balfe, Louise, 70567 Stuttgart (DE); Coffey, Lorcan, 72076 Tuebingen (DE); Unzicker, Kay, 79798 Jestetten (DE); Dzakula, Zdravkica, 8180 Buelach (DE)
(74) Representative: Schmitz, Hans-Werner

(57) **Abstract**

The present invention relates to a catheter for POBA or stent delivery applications. More specifically, the present invention relates to a balloon catheter having a soft distal tip member and methods for manufacturing the same.

## Description

### 1. Priority Claim

The present application claims priority to US Provisional Patent Application Serial Number 60/646,118 filed January 21, 2005, the entirety of which is hereby incorporated by reference.

### 2. Field of the Invention

The present invention relates to a catheter for POBA or stent delivery applications. More specifically, the present invention relates to a balloon catheter having a soft distal tip member.

### 3. Background Information

Non-invasive procedures such as percutaneous transluminal angioplasty (PTA), percutaneous transluminal coronary angioplasty (PTCA), stent delivery and deployment, radiation treatment, delivery of a drug at a lesion site and other procedures are used in the treatment of intravascular disease. These therapies are well known in the art and usually utilize a balloon catheter pulled over a guide wire. After a guiding catheter is placed into the patient's main vessel, a guide wire is advanced in the guide catheter and beyond the distal end of the guide catheter. The balloon catheter is then advanced over the guidewire until it reaches the treatment site at the lesion or stenosis. The balloon is inflated to compress the lesion site and dilate the previous narrowed lesion or stenosis site. If the balloon carried a stent and/or drug, the stent and/or drug is delivered at the site when the balloon is inflated. Likewise, further therapies may also use a balloon catheter for the treatment of the lesion site.

Catheters used in vascular procedures must be flexible and soft to navigate safely through tortuous anatomy of the patient's vessels without damaging the vessels, but at the same time they need sufficient stiffness to allow for good pushability and traceability of the catheter. As a result, catheters have been designed to have a more flexible distal end and a stiffer proximal portion. Particularly with regard to the distal part of the catheter, several ways to achieve a soft tip of the catheter were described in US 4782834, US 4921483, US 5964778, US 2002/0188312, US 2003/0114794, US 2003/0032921, and in US 2003/0139761. However, there continues to be a need for a catheter with an extremely flexible and smooth shaped tip that does not diminish trackability of the catheter and allows the balloon portion of the catheter to smoothly cross the lesion. The present invention addresses this need by providing a novel way to attach a flexible distal tip resulting in a new soft tip member and without diminishing from the trackability and pushability of the catheter.

### 4. SUMMARY OF THE INVENTION

It is an object of the present invention to provide a catheter with a highly flexible and soft tip member at the distal end portion of the catheter.

The invention is directed to a balloon catheter comprising an elongated catheter shaft having a proximal end, a distal end, a proximal shaft section, a distal shaft section, a guide wire lumen extending along at least a distal portion of the catheter shaft to a port at the catheter distal end, and an inflation lumen. The balloon catheter of the present invention further comprises a balloon on the distal catheter shaft section, the balloon having an inflatable interior in fluid communication with the inflation lumen, the distal balloon shaft section bonded to the guide wire lumen tube proximal of the distal end of the guide wire lumen tube and a tip member attached to the outer surface of the distal portion of the guide wire lumen tube and being juxtaposed to the distal end of the balloon shaft section, the tip member extending distally from the balloon shaft and the guide wire lumen tube.

In an alternative embodiment, the tip member is attached to the outer surface of the distal balloon shaft section, the tip member extending distally from the balloon shaft and the guide wire lumen tube. In this embodiment, the guide wire lumen tube can extend up to the distal end of the distal balloon shaft portion or can extend distally to the distal end of the distal balloon shaft portion.

Preferably, the distal tip member is softer than the catheter shaft to provide improved trackability and maneuverability and to decrease the risk of damage to the patient's vessels during advancement therein. In an alternative embodiment, the tip member is formed of or covered with abrasive material to facilitate crossing of stenotic lesions.

The present invention is also directed to a one-step method of forming the tip member generally comprises the steps of positioning the distal balloon shaft over the guide wire lumen, imposing a proximal portion of the soft tip tube on the distal portion of the guide wire lumen tube in a way that the proximal end of the soft tip portion is juxtaposed to the distal end of the balloon cone, and fusion bonding the balloon distal shaft section, the guide wire lumen tube and the soft tip portion to each other. In an alternative embodiment, the proximal portion of the soft tip tube is imposed on the guide wire lumen as well as over a distal end portion of the balloon shaft.

The catheter according to the present invention, having a soft distal tip member attached to the outer surface of the distal portion of the guide wire lumen and being juxtaposed to the distal end of the balloon shaft section or being attached to the outer surface of the distal balloon shaft section as well, shows superior performance with regards to trackability and crossability. With the tip member having a smooth conical shape, the catheter of the present invention has a smooth transition in stiffness and profile from the balloon cone to the distal end of the tip member, thus increasing tensile strength, flexibility and kinking resistance. These and other advantages of the invention will become more apparent from the following detailed description and drawings.

### 5. BRIEF DESCRIPTION OF THE DRAWINGS

Figure 1 is a plan view of an exemplary embodiment of a catheter in accordance with the present invention.
Figure 2A is a cross-sectional view of the distal portion of the catheter illustrating an exemplary embodiment of a tip design in accordance with the present invention.
Figure 2B is a cross sectional view of the distal end of the catheter shown in Figure 2A, taken along line B.
Figure 2C is a cross sectional view of the distal end of the catheter shown in Figure 2A, taken along line C.
Figure 2D is a cross sectional view of the distal end of the catheter shown in Figure 2A, taken along line D.
Figure 3 is a cross-sectional view of the distal portion of the catheter illustrating an alternative tip design.
Figure 4 is a cross-sectional view of the distal portion of the catheter illustrating another alternative tip design.
Figure 5 is a cross-sectional view of the distal portion of the catheter illustrating yet another alternative tip design.
Figure 6 is a cross-sectional view of the distal portion of the catheter illustrating yet another alternative tip design.
Figure 7 is a cross-sectional view of the distal portion of the catheter illustrating yet another alternative tip design.
Figure 8 is a cross-sectional view of the distal portion of the catheter illustrating yet another alternative tip design.
Figure 9 is a cross-sectional view of the distal portion of the catheter illustrating yet another alternative tip design.

### 6. DETAILED DESCRIPTION OF PREFERRED EMBODIMENTS

Reference will now be made in detail to the present preferred embodiments of the invention, an example of which is illustrated in the accompanying drawings. The method and corresponding steps of the invention will be described in conjunction with the detailed description of the system.

The devices and methods presented herein may be used for treating the lumenal systems of a patient. The present invention is particularly suited for treatment of the cardiovascular system of a patient, such as performance of angioplasty and delivery of balloon-expandable or self-expanding interventional devices (e.g., stents, filters, coils).

In accordance with the invention, a catheter is provided including an outer tubular member having a length, an outer surface, an inner surface and a lumen therethrough. The catheter also includes an inner tubular member having an outer surface, an inner surface and a lumen therethrough, at least a length of the inner lumen is disposed in the lumen of the outer tubular member. The catheter further includes an inflatable member disposed adjacent the distal end of the outer tubular member and a distal tip.

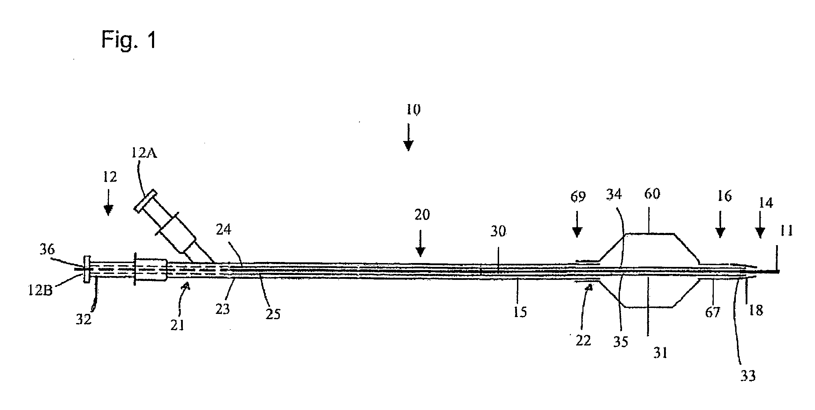

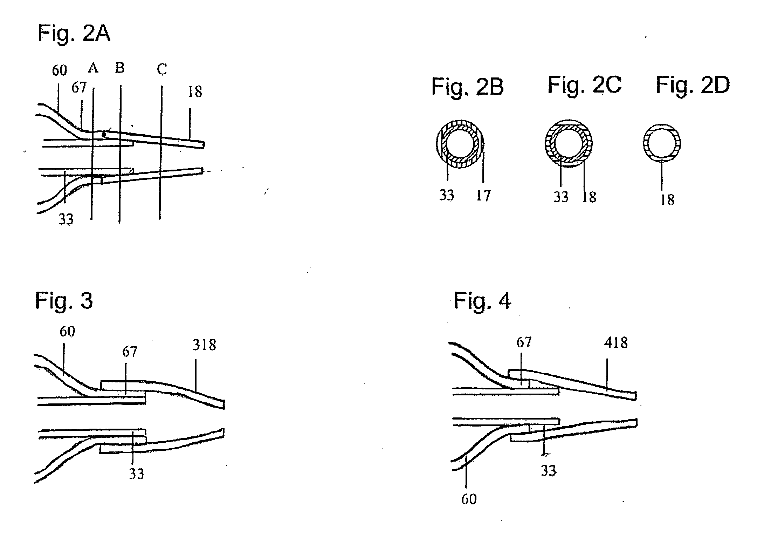

For purpose of explanation and illustration, and not limitation, a side view of an exemplary embodiment of the catheter in accordance with the invention is shown in Fig. 1 and is designated generally by reference character 10. Other embodiments of a catheter in accordance with the invention, or aspects thereof, are provided in Figs. 2-9, as will be described.

For purposes of illustration and not limitation, as embodied herein and as depicted in Fig. 1, catheter 10 is provided with an outer tubular member 20. Outer tubular member 20 has a proximal end 21, a distal end 22, a length L, an outer surface 23, an inner surface 24 and defines a lumen 25 therethrough.

Outer tubular member 20 can be made from a variety of materials, including metal, plastic and composite materials. Metal tubes such as stainless steel hypotubes can be used, and may or may not be coated with a polymeric material such as PTFE. Multilayered polymeric tubes can also be used formed by coextrusion, dipping processes, or by shrinking tubing layers over one another over a mandrel. Moreover, polymeric tubular members can also be formed by charging a mandrel with static electricity, applying plastic in powder or granular form to the mandrel to form a layer of plastic over the mandrel, and by heating the mandrel to cause the particles to fuse. Multilayered polymeric tubes can also be used that include metallic or nonmetallic braiding within or between layers of the tube. A carbon tube can also be used, as well as fiber-reinforced resin materials. If the catheter is only comprised of a single outer tubular along its length, it may be desirable in certain instances to design outer tubular member 20 to have a decreasing stiffness along its length from proximal end 21 to distal end 22.

In further accordance with the invention, a catheter is provided further including an inner tubular member.

For purposes of illustration and not limitation, as embodied herein and as depicted in Fig. 1, catheter 10 includes inner tubular member 30. Inner tubular member 30 has a proximal end 32, a distal end 33, an outer surface 34, an inner surface 35 and defines a lumen 36 therethrough. In accordance with a particular embodiment of the invention depicted in Figure 1, at least a length of the inner tubular member 30 is disposed in the lumen 25 of the outer tubular member 20.

In accordance this aspect of the invention, the inner tubular member 30 can function as a guidewire lumen, as the low friction inner surface 35 of inner tubular member permits a guidewire to move easily through lumen 36. The inner tubular member 30 is disposed within at least a portion of the lumen 25 of the outer tubular member and thereby forming an annular space between the outer surface 34 of the inner tubular member and the inner surface 24 of the outer tubular member 20.

A variety of materials can be used for inner tubular member 30. For example and not limitation, inner tubular member 30 can be made from the same materials as the outer tubular member 20. In accordance with a specific embodiment of the invention, a multilayered tube is used for inner tubular member 30 including a nylon outer layer and an inner layer formed from a lubricious material such as polyethylene of varying densities, PTFE, polyimide, PEEK or PVDF, PEBAX, Nylon, PE, PET, PU and HDPE alone, in blends or in multilayered members. It is further contemplated that the inner tubular member 30 may be constructed of one tube or from two or more composed tube parts, the different parts may consist of different materials.

In a preferred embodiment, the guide wire lumen tube is composed from 2 materials. The proximal portion is formed from a first material, the distal portion of the guide wire lumen tube is formed from a second material more flexible than the first material. Such an arrangement results in a particularly flexible design of the distal end of the guide wire tube and of the catheter. The catheter is highly flexible while still maintaining trackability and pushability. Preferably, the transition portion of the two guide wire lumen tubes is arranged at the proximal portion 69 of the inflatable member 60.

As illustrated in Figure 1, the catheter 10 further includes a hub 12 disposed at the proximal end 21 of the outer tubular member 20, the hub includes a first lumen 12A and a second lumen 12B, wherein the first lumen 12A is in fluid communication with the annular space between the inner surface 24 of the outer tubular member 20 and the outer surface 34 of the inner tubular member 30. The second lumen 12B is in fluid communication with the lumen 36 of the inner tubular member. As shown in Figure 1, a guidewire 11 may be disposed through the second lumen of the hub 12 and through the lumen 36 of the inner tubular member 30, thereby allowing the catheter 20 to be tracked over the guidewire 11 to be placed within a tubular body such as a vessel or artery.

For purposes of illustration and not limitation, as depicted in Fig. 1, the invention, inflation lumen can be used to direct inflation fluid to an inflatable member 60 in fluid communication with the inflation lumen.

Inflatable member 60 can be made from a variety of materials. For purpose of illustration and not limitation, inflatable member 60 can be made from a poly ether block amide ("PEBA"), nylon, Hytrel, PU, PEEK, PE or a variety of other materials. Inflatable member 60 can be attached to distal end 22 of outer tubular member 20 of catheter 10 by way of adhesive bond, fusion bond, or preferably by welding, as described in U.S. Patent Application Serial No. 10/952,543, which is incorporated by reference herein in its entirety. Thus, if inflatable member 60 is made of nylon, it is advantageous for outer tubular member 20 to be made of a material compatible for a welded bond therebetween.

By way of further example, an inflation device (not shown) is provided for inflating the inflatable member 60. The inflation device (not shown) can be, for example, a syringe or a flexible reservoir that is connected to the first lumen 12A of the hub 12 coupled to the proximal end 21 of outer tubular member 20 and actuated by the physician to inflate the inflatable member 60.

As described above, the inflatable member 60 is disposed on the distal end 22 of the outer tubular member 20, wherein the proximal section 69 of the inflatable member 60 is bonded to the outer surface 23 of the outer tubular member 20. The distal section 67 of the inflatable member 60 is bonded to the outer surface 35 of the inner tubular member 30 in the distal end region of the inner tubular member 30.

As shown in Figure 1, the inflatable member 60 spans the length between the distal end 22 of the outer tubular member 20 and the distal end 14 of the catheter 10.

Further in accordance with the present invention, the catheter 10 includes a distal tip member, wherein the distal tip member may be formed in many different configurations as will be shown in Figures 1-8.

Referring now to Figure 2A, there is shown an enlarged longitudinal cross sectional view of one exemplary embodiment of the distal end of the catheter, the tip member 18 as shown in Figure 2A overlaps a portion of the inner tubular member 30 and abuts the distal section 67 of the inflatable member 60, the tip member 18 extending distally from the distal section 67 of the inflatable member 60 and from the distal end 33 of the inner tubular member 30.

The tip member 18 as shown in Figure 2 may be fixedly attached to either or both the distal end portion 67 of the inflatable member and the outer surface 34 of the inner tubular member 30.

Figures 2B, 2C and 2D are cross-sectional views of the distal portion of the catheter 10 illustrated in Figure 1 take about lines B, C and D as shown in Figure 2A.

Figure 2B taken about line B of Figure 2A illustrates the layer formation of the various components of the distal portion. As shown in Figure 2B, the distal portion 69 of the inflatable member 60 is shown disposed radially about the inner tubular member 30.

Figure 2C taken about line C of Figure 2A illustrates the layer formation of the various components of the distal portion. As shown in Figure 2C, the tip member 318 is shown disposed radially about the inner tubular member 30.

Figure 2D taken about line D of Figure 2A illustrates the layer formation of the various components of the distal portion. As shown in Figure 2D, the tip member 318 is the only layer forming the distal tip of the catheter 10.

Referring now to Figure 3, there is shown an alternative exemplary embodiment of a distal tip design 318 end of the catheter. As shown in Figure 3, the alternative tip 318 is formed wherein the distal section 69 of the inflatable member 60 is attached to the distal portion 33 of the inner tubular member 30, the distal end 33 of the inner tubular member 30 extending up to the distal section 69 of the inflatable member 60. The tip portion 318 is fixedly attached to the outer surface of the distal portion 69 of the inflatable member 60, thereby forming a softened tip potion.

Referring now to Figure 4, there is shown yet another exemplary embodiment of an alternative tip design. As illustrated in Fig. 4, the distal section 69 of the inflatable member 60 is fixedly attached to the distal portion 33 of the inner tubular member 30, the distal end 33 of the inner tubular member 30 extending distally from the distal section 69 of the inflatable member 60. The tip portion 418 being fixedly attached to the outer surface of the distal portion 69 of the inflatable tubular member 60 and extends distally from the distal portion 69 of the inflatable member 60 and the inner tubular member 30. In this embodiment, the tip portion 418 is bonded to the outer surface 34 of the distal portion 69 of the inflatable member 60 as well as to the outer surface 34 of the distal portion of the inner tubular member 30 extending distally from the distal portion 69 of the inflatable member 60.

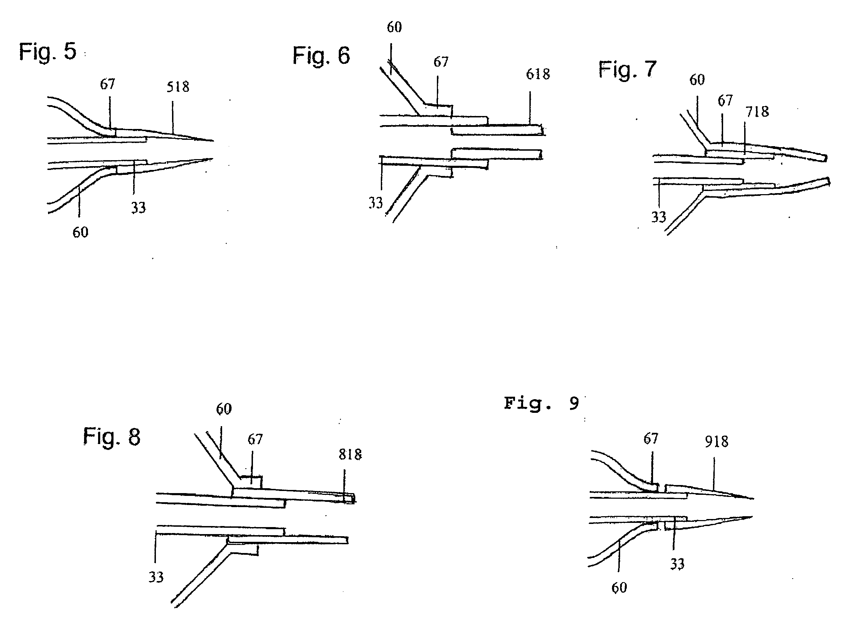

Referring now to Figure 5, there is shown yet another exemplary embodiment of an alternative tip design. As illustrated in Fig. 5, the distal section 69 of the inflatable member 60 is fixedly attached to the distal portion of the inner tubular member 30, the distal end 33 of the inner tubular member 30 extending distally from the distal section 69 of the inflatable member 60. The tip portion 518 being disposed adjacent to and abutting the distal portion 69 of the inflatable member 60 and fixedly attached to the outer surface of the inner tubular member 30.

In yet another preferred embodiment, illustrated in Fig. 6, the distal portion 69 of the inflatable member 60 is affixed to the inner tubular member, wherein a tip member 618 is disposed within and affixed to the inner surface 35 of the inner tubular member 30, wherein the tip member 618 extends beyond the distal end 33 of the inner tubular member 30.

Figure 7 illustrates yet another alternative tip design in accordance with the present invention. As shown in Figure 7, the distal portion of the catheter consists of the distal portion 69 of the inflatable member 60 extending beyond the distal end of the inner tubular member 30. A tip member 718 may be disposed between the distal portion 69 of the inflatable member 60 and the outer surface 34 of the inner tubular member 30 to increase or decrease stiffness, promote bonding or to maintain device profile.

Fig. 8 illustrates yet another exemplary embodiment of a tip formation of the present invention. The tip member 818 is attached to the outer surface 34 of the inner tubular member 30 in a way that the tip member 818 extends distally from the distal end 33 of the inner tubular member 30. The distal portion 69 of the inflatable member 60 is bonded to the outer surface of at least the proximal portion of the tip member 818.

Figure 9 illustrates another exemplary embodiment of an alternative tip design in accordance with the present invention. As shown in Figure 9, the distal section 69 of the inflatable member 60 is fixedly attached to the distal portion of the inner tubular member 30, the distal end 33 of the inner tubular member 30 extending distally from the distal section 69 of the inflatable member 60. The tip portion 918 being disposed adjacent to but not abutting the distal portion 69 of the inflatable member 60, thereby forming a gap between the inflatable member 60 and the proximal portion of the tip 918. The tip member 918 is fixedly attached to the outer surface of the inner tubular member 30. The gap 920 is bridged by a distal portion of the inner tubular member 30 as shown. Thus, a highly flexible portion of the tip portion is formed. Alternatively, the gap 920 may be filled by a metallic material such as platinum, tantalum, gold, silver or similar radiopaque materials to form a marker band. Alternatively, a non-metallic material may be utilized to fill the gap 920 which may be chosen to change the physical properties of the distal portion of the catheter.

In accordance with the present invention and each of the embodiments described in detail above, the tip member of the catheter 10 is preferably formed from a material softer than that of which the inner tubular member is formed in order to provide sufficient flexibility of the catheter tip to allow safe navigation through tortuous anatomy of the patient's vessels but at the same time to provide sufficient pushability and trackability of the catheter to allow crossing of the stenotic vessel sections to be treated.

It is further contemplated though not shown that the tip member may be coated with an abrasive material in order to enable the physician to cross narrow lesions or even total occlusions in the vessels. In another embodiment it is contemplated that the tip material is dyed with a radiopaque material or doped with a radiopaque material to form a tip visible under X-ray.

It would be apparent to the person skilled in the art that the tip member can be made from the same or different material as the balloon and/or the guide wire lumen is made of. Preferably, the tip member in accordance with the present invention is made of PEBAX, Nylon, PE, PET, PU, or blends thereof, or compositions like multilayers, thereof.

In accordance with the present invention herein, it is contemplated that the tip member may be formed in accordance with the methods described herein.

In accordance with one embodiment, the tip and tip portion of the catheter 10 in accordance with the present invention may be constructed in accordance with the following steps: (1) providing an elongated catheter shaft 20 having a proximal end, a distal end, and proximal and distal shaft sections, (2) providing an inner tubular member 30 forming a guide wire lumen 36 extending along at least a distal portion of the catheter shaft 20, wherein an annular space between the catheter shaft and the inner tubular member forms an inflation lumen, (3) providing an inflatable member 60 disposed on a distal portion of the catheter 10 and in fluid communication with the inflation lumen. (4) Positioning a tip member 18 at the outer surface 34 of the distal portion of the inner tubular member 30 at a position juxtaposed to the distal end of the distal portion 69 of the inflatable member 60 in such a way that the tip member 18 extends distally from the distal portion 69 of the inflatable member 60 and the distal end 33 of the inner tubular member 30. (5) Forming the tip portion by bonding the tip member 18, the distal portion 69 of the inflatable member 60 and the outer surface 34 of the inner tubular member 30 together.

In accordance with the present invention, the forming procedure, described above will be described in greater detail below, wherein the tip member is attached to the catheter assembly in accordance with the following procedures. The inner tubular member 30 is arranged or suited in a way that it extends a relatively short distance of approx. 0.1 to 10 mm, preferably 1 mm distal of the distal portion 69 of the inflatable member 60. A proximal portion of the tip member 18 is pulled over the distal portion of the inner tubular member 30 to be juxtaposed to the distal portion 69 of the inflatable member 60. The tip portion 18 and the distal portion 69 of the inflatable member 60 are fusion bonded in a one step process to the outer surface 34 of the inner tubular member 30 by applying heat to at least a portion of the proximal tip portion and the distal portion 69 of the inflatable member 60, to form a catheter tip wherein the tip member 18 is attached to the outer surface 34 of the distal portion of the inner tubular member 30 at a position juxtaposed to the distal portion 69 of the inflatable member 60 in such a way that the tip member 18 extends distally from the distal portion 69 of the inflatable member 60 and the inner tubular member 30.

In an alternative embodiment the tip member 18 is positioned at at least a portion of the distal portion 69 of the inflatable member 60, the tip member 18 extending distally from the distal portion 69 of the inflatable member 60 and the inner tubular member 30 to form the tip portion, the tip member, the distal portion 69 of the inflatable member 60 and the inner tubular member 30 are bonded to each other. In this embodiment, the guidewire lumen 36 can extend distally of the inflatable member 60 as shown in Fig. 4, resulting in attachment of the tip member to the outer surface 34 of the distal portion 69 of the inflatable member 60 and the outer surface 34 of the inner tubular member 30. In the alternative design as shown in Fig. 3, the tip member 18 is merely attached to the outer surface 34 of the distal portion 69 of the inflatable member 60, while the distal portion 69 of the inflatable member 60 is attached to the outer surface 34 of the inner tubular member 30.

In order to attach the tip member 18 to the catheter assembly in this alternative embodiment, the inner tubular member 30 is arranged in the desired position relatively to the distal portion 69. A proximal portion of the tip member 18 is pulled over the distal portion of the inner tubular member 30 and at least a portion of the distal portion 69 of the inflatable member 60. The tip member 18, the distal portion 69 of the inflatable member 60 and the inner tubular member 30 are fusion bonded in a one step process by applying heat to at least a portion of the proximal tip portion and the distal portion 69 of the inflatable member 60, to form a balloon catheter tip wherein the tip member is attached to the outer surface of the distal portion 69 of the inflatable member 60.

In accordance with an alternative embodiment of a method for manufacturing a balloon catheter 10 as illustrated in Fig. 6, an elongated catheter shaft 20 is provided, having a proximal end, a distal end, a proximal shaft section, a distal shaft section, an inner tubular member 30 having a guidewire lumen 36 extending along at least a distal portion of the catheter shaft 20 to a port at the catheter distal end, and an inflation lumen. Further, an inflatable member such as a balloon 60 is provided on the distal catheter shaft section, the balloon 60 having an inflatable interior in fluid communication with the inflation lumen. The distal balloon shaft section 69 is positioned about the inner tubular member 30 in a distal end region of the inner tubular member, the tip member 18 is positioned along a distal portion of the inner surface 35 of the distal portion of the inner tubular member 30, in a way that the tip member 18 extends distally from the balloon shaft 69 and the inner tubular member 30. The tip portion 18 is then formed in a one step bonding process by bonding the tip member 18 and the balloon shaft section 69 to the outer surface 34 of the inner tubular member 30.

In an alternative embodiment of a method for manufacturing the tip portion of a balloon catheter 10 as illustrated in Fig. 8, a tip member 18 is positioned at the distal portion of the outer surface of the inner tubular member 30, such that the tip member 18 extends distally from the distal end of the inner tubular member 30. The distal balloon shaft section 69 is positioned along a proximal portion of the tip member 18. Finally, the tip portion is formed by bonding the tip member 18 to the balloon shaft section 69 and the inner tubular member 30. In an alternative embodiment, the balloon shaft section 69 may extend distally of the tip member 18 and forms the distal tip of the catheter 10 as illustrated in Fig. 7.

Typically, for all of the methods of forming the tip portion described above, a mandrel is inserted into the distal guide wire tube and the tip member before fusion bonding is performed. Optionally, a shrink tube can be imposed on the distal balloon shaft and the tip member to achieve good heat distribution and a defined final outer diameter of the tip portion. Both, the mandrel and the shrink tube will be removed after the bonding process is finished. The fusion bonding can be performed by various welding processes including but not limited to contact welding, hot air welding, laser welding, inductive welding, and white light welding technology (shown and described in co-pending US Patent Application serial number 10/952,543 the entirety of which is hereby incorporated by reference. In a preferred embodiment, the fusion bonding is done by transition bonding.

The methods of forming the tip portion of the catheter of the present invention allow the formation of an extremely smooth transition of the balloon cone to the distal tip having a soft taper of the tip portion. Further, all methods allow the formation of the tip portion in a one step process, thus, no successive bonding steps are needed.

One skilled in the art would readily appreciate that the present invention is well adapted to carry out the objects and obtain the ends and advantages mentioned, as well as those inherent therein. The methods, processes, and devices described herein are presently representative of preferred embodiments, are exemplary, and are not intended as limitations on the scope of the invention. It will be readily apparent to one skilled in the art that varying substitutions and modifications may be made to the invention disclosed herein without departing from the scope and spirit of the invention.

All patents and publications mentioned in the specification are indicative of the levels of those skilled in the art to which the invention pertains. All patents and publications are herein incorporated by reference to the same extent as if each individual publication was specifically and individually indicated to be incorporated by reference.

The catheter 10 shown in Figure 1 and described herein is an over-the-wire type catheter. However, the catheter of the present invention may be a rapid exchange catheter as well, wherein the proximal guide wire lumen port is located at a position distal of the proximal end of the catheter. In one embodiment of the present invention the guide wire lumen and inflation lumen are arranged coaxially to each other. However, the guide wire lumen and inflation lumen can be arranged side-by-side as well.

The invention illustratively described herein suitably may be practiced in the absence of any element or elements, limitation or limitations which is not specifically disclosed herein. Thus, for example, in each instance herein any of the terms "comprising", "consisting essentially of" and "consisting of" may be replaced with either of the other two terms. The terms and expressions which have been employed are used as terms of description and not of limitation, and there is no intention that in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the concepts herein disclosed may be resorted to by those skilled in the art, and that such modifications and variations are considered to be within the scope of this invention as defined by the appended claims.

The method according to the present invention can provide that the tip portion is formed by heat welding or by laser welding.

Furthermore, the inventive method can provide that a mandrel is inserted into the guidewire lumen of the inner tubular member and the tip member before bonding is performed.

Moreover, in accordance with the method according to the present invention, a shrink tube can be imposed on the distal balloon shaft and the tip member before bonding is performed.

Furthermore, the tip portion can be formed by white light welding and the inner tubular member and the outer tubular member can be co-extruded.

Finally, the method according to the present invention can include the step of providing a luer, the luer attached to the proximal end of the outer tubular member and to the proximal end of the inner tubular member, the luer further including a first lumen and a second lumen, the first lumen being in fluid communication with the balloon, and the second being in communication with the guidewire lumen.

## Claims

1. A balloon catheter comprising:
an outer tubular member having a proximal end, a distal end, a proximal shaft section, a distal shaft section;
an inner tubular member extending within at least a distal portion of the outer tubular member, and spaced apart from at least a portion of the outer tubular member to form an inflation lumen therebetween;
a balloon disposed adjacent the distal end of the cathether and having an inflatable interior in fluid communication with the inflation lumen, a proximal balloon shaft section coupled to the distal portion of the outer tubular member, a distal balloon shaft section being bonded to the inner tubular member; and
a tip member extending from a distal end of the inner tubular member, the tip member associated with the distal balloon shaft section.

2. The balloon catheter according to claim 1, wherein a proximal portion of the tip member abuts the distal balloon shaft section.

3. The balloon catheter according to claim 1, wherein a portion of the tip member is disposed radially about the distal balloon shaft section, the distal balloon shaft section affixed to the distal end of the inner tubular lumen.

4. The balloon catheter according to claim 1, wherein the tip member is fixedly attached to an inner lumen of the inner tubular member and the distal balloon section is affixed to an outer surface of the inner tubular member.

5. The balloon catheter according to claim 3, wherein the distal balloon shaft section extends distally beyond the tip member.

6. The balloon catheter according to claim 1, wherein a proximal portion of the tip member is disposed adjacent to the distal balloon shaft section, thereby forming a space therebetween.

7. The balloon catheter according to claim 6, wherein a distal portion of the catheter is more flexible than a proximal portion of the cathether.

8. The balloon catheter according to claim 1, wherein the tip member is constructed of a material chosen from the group consisting of PEBAX, Nylon, PE, PET, PU, blends thereof, compositions, and multilayers thereof.

9. A balloon catheter comprising:
an outer tubular member having a proximal end, a distal end, a proximal shaft section, a distal shaft section;
an inner tubular member extending within at least a distal portion of the outer tubular member, and spaced apart from at least a portion of the outer tubular member to form an inflation lumen therebetween, and having a guidewire lumen extending therethrough;
a balloon disposed adjacent the distal end of the cathether and having an inflatable interior in fluid communication with the inflation lumen, a proximal balloon shaft section coupled to the distal portion of the outer tubular member, a distal balloon shaft section being bonded to the inner tubular member; and
a tip member attached to an outer surface of the inner tubular member and being juxtaposed to a distal end of the distal balloon shaft section, wherein the distal balloon shaft portion extends distally of the tip member and forms the distal tip of the catheter.

10. The catheter according to claim 9, wherein an inner diameter of the tip member tapers down to a diameter substantially equal to an inner diameter of the inner tubular member.

11. The catheter according to claim 9, wherein the material of the tip member is softer than the material of the inner tubular member.

12. The catheter according to claim 9, wherein the inflation lumen and the guide wire lumen are arranged coaxially to each other.

13. The catheter according to claim 9, wherein the inflation lumen and the guide wire lumen are arranged side-by-side to each other.

14. A method for manufacturing a balloon catheter, the method comprising the steps of:
providing an elongated outer tubular member having a proximal end, a distal end, a proximal shaft section, a distal shaft section;
providing an inner tubular member extending along and throuhg at least a distal portion of the catheter shaft to a port at the distal end of the outer tubular member and forming an inflation lumen therebetween;
providing a balloon having a proximal shaft section and a distal shaft section, the proximal shaft section affixed to the distal end of the outer tubular member and having an inflatable interior in fluid communication with the inflation lumen, the distal balloon shaft section affixed to an outer surface of the inner tubular member;
positioning a tip member at the outer surface inner tubular member at a position juxtaposed to a distal end of the distal balloon shaft section in such a way that the tip member extends distally from the balloon shaft and the guide wire lumen tube; and
forming the tip portion by bonding the tip member, the balloon shaft section and the inner tubular member to each other.
